(19) **Europäisches Patentamt**
**European Patent Office**
**Office européen des brevets**

(11) **EP 4 092 007 A1**

(12) **EUROPÄISCHE PATENTANMELDUNG**

(43) Veröffentlichungstag:
**23.11.2022 Patentblatt 2022/47**

(21) Anmeldenummer: **21174713.4**

(22) Anmeldetag: **19.05.2021**

(51) Internationale Patentklassifikation (IPC):
**C04B 35/111** (2006.01)    **C04B 35/16** (2006.01)
**C04B 35/443** (2006.01)    **C04B 35/486** (2006.01)
**C04B 35/626** (2006.01)    **A61C 13/00** (2006.01)
**C03C 10/00** (2006.01)

(52) Gemeinsame Patentklassifikation (CPC):
**C04B 35/16; C03C 1/10; C03C 3/076;**
**C03C 4/0021; C03C 4/02; C03C 10/0027;**
**C04B 35/111; C04B 35/443; C04B 35/486;**
**C04B 35/6263; C04B 35/6264; C04B 35/62802;**
A61C 13/0022; C03C 2205/06; C04B 2235/3201;

(Forts.)

(84) Benannte Vertragsstaaten:
**AL AT BE BG CH CY CZ DE DK EE ES FI FR GB**
**GR HR HU IE IS IT LI LT LU LV MC MK MT NL NO**
**PL PT RO RS SE SI SK SM TR**
Benannte Erstreckungsstaaten:
**BA ME**
Benannte Validierungsstaaten:
**KH MA MD TN**

(71) Anmelder: **Vita Zahnfabrik H. Rauter GmbH & Co. KG**
**79713 Bad Säckingen (DE)**

(72) Erfinder:
• **OBERLÄNDER, Andreas**
  **79713 Bad Säckingen (DE)**

• **GÖDIKER, Berit**
  **79713 Bad Säckingen (DE)**
• **BOJEMÜLLER, Enno**
  **79713 Bad Säckingen (DE)**
• **STRASSER, Christian**
  **79713 Bad Säckingen (DE)**
• **HELLMEHL, Eduard**
  **79713 Bad Säckingen (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(54) **VERFAHREN ZUR HERSTELLUNG EINES ROHLINGS FÜR DENTALE RESTAURATIONEN MITTELS EINSTUFIGEM SEDIMENTATIONSVERFAHREN**

(57)    Verfahren zur Herstellung eines Rohlings für dentale Restaurationen mit Farbverlauf umfassend die Schritte:

   a) Bereitstellen einer Suspension umfassend

   i) flüssiges Dispersionsmittel,

   ii) ein Basismaterial und

   iii) ein oder mehrere färbende Substanzen;

   b) Sedimentation unter Ausbildung eines Farbgradienten im Sediment

   c) Verfestigen des Sediments unter Ausbildung eines Rohlings mit Farbverlauf.

   sowie Verwendung der Suspension und eine Rohling resultierend aus dem Verfahren. Das Basismaterial ist ausgewählt ist aus der Gruppe bestehend aus Silikat-Glaskeramiken und -Gläsern, Aluminiumoxide, Spinelle und Zirkoniumoxide.

Figur 1

**(Forts. nächste Seite)**

EP 4 092 007 A1

(52) Gemeinsame Patentklassifikation (CPC): (Forts.)
C04B 2235/3206; C04B 2235/3208;
C04B 2235/3217; C04B 2235/3224;
C04B 2235/3225; C04B 2235/3229;
C04B 2235/3232; C04B 2235/3241;
C04B 2235/3244; C04B 2235/3262;
C04B 2235/3272; C04B 2235/3275;
C04B 2235/3279; C04B 2235/3284;
C04B 2235/3293; C04B 2235/3294;
C04B 2235/3418; C04B 2235/5436;
C04B 2235/5463; C04B 2235/5481;
C04B 2235/616; C04B 2235/75; C04B 2235/9661

**Beschreibung**

Die vorliegende Anmeldung betrifft ein Verfahren zur Herstellung eines Rohlings mit Farbverlauf für dentale Restaurationen mittels Sedimentationsverfahren, ein mittels des erfindungsgemäßen Verfahrens gewonnenen Rohling sowie die Verwendung einer Suspension zur Herstellung einer dentalen Restauration mit Farbverlauf.

Unter dem Begriff der dentalen Restauration werden Maßnahmen verstanden, defekte Zähne wiederherzustellen beziehungsweise verlorengegangene Zähne zu ersetzen. Dies kann beispielsweise in Form von festsitzendem Zahnersatz wie Kronen, Teilkronen, Brücken oder Implantaten oder in Form von herausnehmbarem Zahnersatz wie Voll- oder Teilprothesen geschehen. Diesen Maßnahmen ist gemein, dass die verwendeten Restaurationen dem Aussehen natürlicher Zähne möglichst nahekommen sollen, um ein ästhetisch ansprechendes Bild zu erzeugen.

Um eine ästhetisch ansprechende Restauration zu erreichen, die sich in das natürliche Zahnumfeld eines Patienten einfügt, kann die Restauration entsprechend eingefärbt werden, beispielsweise durch Bemalen. Alternativ können zur Herstellung dentaler Restaurationen Rohlinge verwendet werden, die bereits über einen entsprechenden Farbverlauf verfügen, der dem des natürlichen Zahns nachempfunden ist. Um diesen Farbverlauf zu erreichen, wird der Rohling meist aus mehreren Schichten aufgebaut, die sich hinsichtlich ihrer Farbgebung unterscheiden.

WO 2020/025795 beschreibt einen Rohling für eine dentale Restauration, der eine erste und eine zweite Schicht aufweist, die unabhängig voneinander aus einem Glas, einer Glaskeramik oder einer Keramik aufgebaut sind, wobei die erste Schicht und die zweite Schicht sich hinsichtlich ihrer Farbe unterscheiden und eine Grenzfläche bilden, die innerhalb des Rohlings in einem Winkel angeordnet ist.

DE 197 14 178 betrifft ein Verfahren zur Herstellung eines mehrfarbigen Formkörpers für die Weiterverarbeitung zu Zahnrestaurationen, bei dem mindestens zwei unterschiedlich gefärbte Ausgangsmaterialien in eine, im Wesentlichen die Form des Formkörpers vorgebende, Pressmatrize eingefüllt und zu einem Formkörper verpresst werden.

EP 1 900 341 offenbart einen mehrfarbigen Formkörper mit aufeinander gelagerten Schichten zur Herausarbeitung von Dentalrestaurationen, der (a) mindestens zwei aufeinander folgende und unterschiedlich gefärbte Hauptschichten und (b) zwischen mindestens zwei aufeinander folgenden und unterschiedlich gefärbten Hauptschichten mindestens zwei unterschiedlich gefärbte Zwischenschichten aufweist, wobei die Hauptschichten eine Dicke von mehr als 0,5 mm haben und die Zwischenschichten eine Dicke von 0,1 bis 0,5 mm haben und wobei zwischen den Zwischenschichten eine Änderung der Farbe in einer Richtung erfolgt, die der Richtung der Änderung der Farbe zwischen den Hauptschichten entgegengesetzt ist.

Gemäß EP 3 215 820 umfasst ein computerimplementiertes Verfahren zum Herstellen einer Zahnrestauration die Schritte: Erfassen eines Bilds eines Zahns bei einer Farbtiefe, die auf einer Multiplizität von Farbwerten basiert; Positionieren des Bilds durch Erkennen, in dem Zahnbild, eines zusammenhängenden ersten Zahnfarbbereichs mit Farbwerten innerhalb eines vorbestimmten ersten Bereichs von unterschiedlichen Farbwerten und Zuordnen des ersten Zahnfarbbereichs zu einem gemeinsamen ersten Falschfarbwert; und Erkennen, in einem Zahnbild, eines zusammenhängenden zweiten Zahnfarbbereichs mit Farbwerten innerhalb eines vorbestimmten zweiten Bereichs von unterschiedlichen Farbwerten und Zuordnen zu einem gemeinsamen zweiten Falschfarbwert; Bestimmen einer Zahnfarbstruktur basierend auf dem ersten und dem zweiten Falschfarbwert des ersten beziehungsweise des zweiten Farbbereichs innerhalb des Zahnbilds; Bereitstellen von Informationen über einen mehrfarbigen Block, der eine vorbestimmte Farbschattierung aufweist, gebildet durch mindestens eine erste Blockfarbzone und eine zweite Blockfarbzone, wobei die Informationen Daten über eine Blockfarbstruktur in Bezug auf Abmessungen und/oder Positionen der ersten und der zweiten Blockfarbzone umfassen; Abgleichen der Zahnfarbstruktur mit der Blockfarbstruktur; und basierend auf dem Abgleich; Bestimmen einer Position innerhalb des Blocks, in der die Zahnfarbstruktur und die Blockfarbstruktur innerhalb vorbestimmter Grenzen übereinstimmen; und maschinelles Bearbeiten der Zahnrestauration von dem Block in der bestimmten Position.

EP 3 527 166 beschreibt einen Fräsrohling aus Kunststoff oder einem kunststoffbasierten mit einem kontinuierlichen Farbverlauf zur Herstellung einer indirekten dentalen Restauration und ein Verfahren zur Herstellung eines solchen Rohlings durch Mischen zweier unterschiedlich gefärbter Pasten unter kontinuierlicher Variation des Mischungsverhältnisses der beiden Pasten während des Abfüllvorgangs.

Den im Stand der Techniken bekannten Verfahren und Techniken zum Herstellen ästhetisch ansprechender dentaler Restaurationen ist gemein, dass die Farbgestaltung sehr aufwendig ist und teilweise geschultes Personal mit langjähriger Erfahrung benötigt wird, um zufriedenstellende Ergebnisse zu erzielen. In den Fällen, in denen die Restaurationen ausgehend von entsprechend eingefärbten Rohlingen gefertigt werden, besteht das Problem, dass der für die Erzielung des Farbverlaufs notwendige schichtweise Aufbau oft dazu führt, dass die unterschiedlichen Schichten auch in der fertigen Restauration zu erkennen sind und so ein fließender Übergang zwischen den einzelnen Farbschichten nicht erreicht werden kann.

Es besteht daher weiterhin der Bedarf nach Verfahren, die die Herstellung von ästhetisch ansprechenden dentalen Restaurationen erlauben. Daher ist es die Aufgabe der vorliegenden Erfindung, ein Verfahren zur Verfügung zu stellen, mit dem dentale Restaurationen zugänglich sind, deren optisches Erscheinungsbild dem eines natürlichen Zahns entspricht und entsprechend den Bedürfnissen des Patienten angepasst werden kann.

Es wurde überraschend gefunden, dass diese Aufgabe durch ein Verfahren gelöst wird, bei dem der Rohling mittels Sedimentationsverfahren gewonnen wird.

Das Sedimentationsverhalten von in einer Suspension enthaltenen Festoffen kann, wie dem Fachmann bekannt ist, anhand der Stokes-Gleichung dargestellt werden:

$$v_p = \frac{2}{9} * \frac{r^2 g (\rho_p - \rho_f)}{\eta} \text{,}$$

mit

$v_p$: Sedimentationsgeschwindigkeit

r: Radius des sinkenden Gegenstands

g: Erdbeschleunigung

$\rho_p$: Dichte des Partikels

$\rho_f$: Dichte des Fluids

$\eta$: dynamische Viskosität des Fluids

Im Rahmen der vorliegenden Erfindung wurde überraschend gefunden, dass dieses allgemeine Prinzip auch bei der Herstellung von dentalen Restaurationen mittels sedimentationsbasierten Formgebungsprozessen zugrunde gelegt werden kann, um Rohlinge mit einem stufenlosen Farbverlauf zu erhalten.

Daher ist ein erster Gegenstand der vorliegenden Anmeldung ein Verfahren zur Herstellung eines Rohlings für dentale Restaurationen mit Farbverlauf umfassend die Schritte:

a) Bereitstellen einer Suspension umfassend

i) flüssiges Dispersionsmittel,

ii) ein Basismaterial und

iii) ein oder mehrere färbende Substanzen;

b) Sedimentation der Suspension unter Ausbildung eines Sediments mit Farbgradient;

c) Verfestigen des Sediments; unter Ausbildung eines Rohlings mit Farbverlauf.

Es hat sich überraschend gezeigt, dass mittels des erfindungsgemäßen Verfahrens Rohlinge erhalten werden, die den natürlichen Farbverlauf eines Zahns abbilden, ohne dass Farbübergänge zu erkennen wären wie sie beispielsweise bei Rohlingen auftreten, die mittels Schichtaufbau hergestellt werden. Ohne an eine bestimmte Theorie gebunden zu sein, wird davon ausgegangen, dass die stufenlose Ausbildung des Farbverlaufs insbesondere durch das unterschiedliche Sedimentationsverhalten der in der Suspension enthaltenen Partikel zustande kommt.

Als weiterer Vorteil des erfindungsgemäßen Verfahrens hat sich dessen breite Anwendbarkeit auf unterschiedliche Materialien herausgestellt. So können als Basismaterial alle im Bereich der dentalen Restaurationen üblichen Materialien eingesetzt werden. Daher ist eine Ausführungsform bevorzugt, in der das Basismaterial ausgewählt ist aus der Gruppe bestehend aus Silikat-Glaskeramiken und Silikat-Gläsern, Aluminiumoxiden, Spinelle und Zirkoniumoxiden.

In einer besonders bevorzugten Ausführungsform ist das Basismaterial ausgewählt aus der Gruppe bestehend aus Silikat-Glaskeramiken und Silikat-Gläsern, insbesondere Feldspate, Foide, Lithiumsilikate und Leucit-Glaskeramiken. In einer weiterhin bevorzugten Ausführungsform ist das Basismaterial gefärbt.

Gemäß dem erfindungsgemäßen Verfahren wird der Farbverlauf im Rohling durch die Zugabe färbender Substanzen erreicht, wobei diese je nach Bedarf zusammengestellt werden können. In einer bevorzugten Ausführungsform handelt es sich bei den färbenden Substanzen um Farbpigmente, vorzugsweise um Oxide von Elementen, die ausgewählt sind aus Zr, Hf, Zn, Ti, Fe, Al, Si, Cr, Sb, Mn, Cd, Sn, Ca, Na, K, Mg, Ni, Co, Sc, Y, Ce, Er, Yb, Pr, Eu, Dy, Tb und Mischungen hiervon.

Die Farbpigmente können in der Suspension in unterschiedlicher Form vorliegen. So ist eine Ausführungsform bevorzugt, in der die Farbpigmente in kolloidaler Form, Pulverform oder als Fritte vorliegen.

Die Farbgebung des Rohlings kann durch unterschiedliche Techniken erreicht werden. In einer bevorzugten Ausführungsform liegen das Basismaterial und die färbenden Substanzen als Gemisch in der Suspension vor.

Im Rahmen der vorliegenden Erfindung wurde überraschend festgestellt, dass durch Modifizieren der Oberfläche der färbenden Substanzen deren Sedimentationsverhalten beeinflusst und so der Farbverlauf des Rohlings gesteuert werden kann. So können die färbenden Substanzen beispielsweise verkapselt werden. Daher ist alternativ eine Ausführungsform bevorzugt, in der als färbende Substanzen oberflächenmodifizierte Pigmente eingesetzt werden. Die Oberflächenmodifikation kann beispielsweise dadurch erreicht werden, dass die färbenden Substanzen in Gegenwart eines geeigneten Verkapselungsmaterials einer thermischen Behandlung unterzogen werden, bei der das Verkapselungsmaterial aufgeschmolzen wird und so die intakt gebliebenen Pigmente umschließt.

In einer weiterhin bevorzugten Ausführungsform liegen das Basismaterial und die färbenden Substanzen in integrierter Form, beispielsweise als Fritte vor. Ein solches Material kann beispielsweise dadurch erhalten werden, dass das Basismaterial zusammen mit der oder den färbenden Substanzen aufgeschmolzen wird und die so erhaltene Fritte durch Mahlen auf die gewünschte Partikelgröße gebracht wird.

Die Entstehung des Farbverlaufs im gemäß dem erfindungsgemäßen Verfahren erhältlichen Rohling wird insbesondere durch das unterschiedliche Sedimentationsverhalten der Bestandteile der Suspension bestimmt, das wiederum durch die Wahl eines geeigneten Dispersionsmittels beeinflusst werden kann. Insofern ist eine Ausführungsform bevorzugt, in der das Dispersionsmittel ausgewählt ist aus der Gruppe bestehend aus Wasser, organischen Lösungsmitteln, insbesondere Alkoholen, flüssigen polymerisierbaren Monomeren und Mischungen davon.

Das Sedimentationsverhalten von in einer Suspension enthaltenen Feststoffen wird unter anderem vom Feststoffgehalt der Suspension beeinflusst. Im Rahmen des erfindungsgemäßen Verfahrens hat es sich als besonders vorteilhaft herausgestellt, wenn der Feststoffgehalt der verwendeten Suspension nicht mehr als 70 Vol.-%, bezogen auf das Gesamtvolumen der Suspension beträgt, da auf diese Weise ein übergangsloser Farbverlauf im Rohling erhalten werden kann. Daher ist eine Ausführungsform bevorzugt, in der die Suspension einen Feststoffgehalt von 15 bis 65 Vol.-%, vorzugsweise 35 bis 60 Vol.-% aufweist, jeweils bezogen auf das Gesamtvolumen der Suspension.

Der Farbverlauf im erfindungsgemäß erhaltenem Rohling kann weiterhin durch die Dichte der in der Suspension enthaltenen Partikel bestimmt werden. Dabei hat es sich vorteilhaft erwiesen, wenn das Basismaterial eine von der Dichte der färbenden Substanzen abweichende Dichte aufweist, da auf diese Weise der natürliche Farbverlauf eines Zahns nachempfunden werden kann. Daher ist eine Ausführungsform bevorzugt, in der das Basismaterial eine von der Dichte der färbenden Substanzen abweichende Dichte aufweist. Vorzugsweise ist die Dichte des Basismaterials geringer als die Dichte der färbenden Substanzen. In einer weiterhin bevorzugten Ausführungsform weist das Basismaterial eine Dichte von 2 bis 6 g/cm$^3$, vorzugsweise 2 bis 3 g/cm$^3$ auf. Die ein oder mehreren färbenden Substanzen, die in der Suspension eingesetzt werden, weisen vorzugsweise eine Dichte von 3 bis 13 g/cm$^3$, besonders bevorzugt von 4 bis 8 g/cm$^3$ auf. Die Dichte kann dabei beispielsweise mittels Helium-Pyknometrie bestimmt werden.

Ein weiterer Faktor, der zur Beeinflussung des Sedimentationsverhaltens herangezogen werden kann, ist die Partikelgröße der in der Suspension enthaltenden Bestandteile. Im Rahmen der vorliegenden Erfindung hat sich überraschend gezeigt, dass durch die Auswahl von Basismaterial und färbenden Substanzen mit geeigneten Partikelgrößen vermieden werden kann, dass sich sichtbare Übergänge zwischen den unterschiedlich gefärbten Bereichen des Rohlings ausbilden. Daher ist eine Ausführungsform bevorzugt, in der das Basismaterial und/oder die färbenden Substanzen eine durchschnittliche Partikelgröße D50 von 0,03 bis 100 μm, vorzugsweise 1 bis 20 μm aufweisen, bestimmt mittels statischer und/oder dynamischer Lichtstreuung. Der D50-Wert der Partikelgröße bezeichnet dabei die Anzahl der Partikel, die größer und kleiner als der angegebene Wert sind und wird daher auch als durchschnittliche oder mittlere Partikelgröße bezeichnet.

Die Partikelgrößenverteilung eines beliebigen pulverförmigen Materials wird in der Regel mittels der Werte D10, D50 und D90 angegeben, wobei D10 die 10% der Partikel bezeichnet, die eine Partikelgröße aufweisen, die kleiner als der angegebene Wert ist, D50 die durchschnittliche Partikelgröße angibt und D90 die 90% der Partikel bezeichnet, die eine Partikelgröße aufweisen, die unterhalb des angegebenen Werts liegt. Die %-Angaben beziehen sich dabei auf das Gesamtvolumen der Partikel.

Das erfindungsgemäße Verfahren zeichnet sich in einer bevorzugten Ausführungsform dadurch aus, dass das Basismaterial eine Partikelgrößenverteilung aufweist, in der der D10-Wert ein Fünftel, vorzugsweise die Hälfte des D50-Werts beträgt. Weiterhin beträgt der D90-Wert der Partikelgrößenverteilung das Vierfache, vorzugsweise das Doppelte des D50-Werts. Die Partikelgrößenverteilung des Basismaterials ist vorzugsweise ausgedrückt als ein D10-Wert von 0,2*D50, vorzugsweise 0,5*D50 und einem D90-Wert von 4*D50, vorzugsweise 2*D50.

Die im erfindungsgemäßen Verfahren eingesetzten färbenden Substanzen zeichnen sich vorzugsweise durch folgende Partikelgrößenverteilung aus: D10 von 0,1*D50, vorzugsweise 0,4*D50, und D90 von 5*D50, vorzugsweise 2*D50. Auf diese Weise konnte ein natürlicher Farbverlauf des Rohlings erreicht werden, ohne dass es zu einer übermäßigen Farbintensität im unteren Bereich des Rohlings kommt.

Der angestrebte Farbverlauf im Rohling konnte weiterhin dadurch optimiert werden, dass der Suspension weitere Bestandteile zugesetzt wurden. Daher ist eine Ausführungsform des erfindungsgemäßen Verfahrens bevorzugt, in der die Suspension weiterhin Additive umfasst, vorzugsweise Netzmittel und/oder Dispergatoren. Das Netzmittel ist dabei vorzugsweise ausgewählt aus der Gruppe der nichtionischen Tenside. Der Dispergator ist vorzugswiese ausgewählt aus der Gruppe bestehend aus Wasserglas, Polyelektrolyte, Tenside, Carbonsäureester, Amine, Aminalkohole, Polycarbonate, Polycarbonsäurederivate, Silane, Silikat-Polycarbonate und Mischungen hiervon.

Der durch das erfindungsgemäße Verfahren erhaltene Rohling ist als dentale Restauration vorgesehen, wobei es sich als vorteilhaft erwiesen hat, die Formgebund des Rohlings dadurch zu erreichen, dass die Suspension in entsprechende Gießformen gefüllt wird. Daher ist insbesondere bei der Viskosität der Suspension darauf zu achten, dass zum einen der gewünschte Farbverlauf erreicht wird und zum anderen eine blasenfreie Füllung der Gießform erfolgt. Daher ist eine Ausführungsform bevorzugt, in der die Suspension eine Viskosität von 1 mPas bis $10^5$ mPas, vorzugsweise 1 mPas bis $10^3$ mPas, bestimmt bei 25 °C mittels eines Rheometers (z.B. Physica MCR 101 Rheometer von Anton Paar), aufweist.

Das erfindungsgemäße Verfahren sieht das Sedimentieren der bereitgestellten Suspension vor. Unter Sedimentieren im Sinne der vorliegenden Erfindung wird das Ablagern beziehungsweise Absetzen der in der Suspension enthaltenen Feststoffe verstanden. Dabei setzten sich die Partikel abhängig von ihrer Sedimentationsgeschwindigkeit ab, wobei die Partikel mit der größten Sedimentationsgeschwindigkeit sich zuerst absetzen, also im erfindungsgemäß erhaltenem Rohling zuunterst angeordnet sind.

Die Sedimentation kann in einer bevorzugten Ausführungsform mittels Zentrifugalkraft bewirkt werden.

Schritt c) des erfindungsgemäßen Verfahrens sieht die Verfestigung des in Schritt b) erhaltenen Sediments vor. Diese Verfestigung kann beispielsweise mittels Trocknen erfolgen oder, in Fällen, in denen polymerisierbare Monomere als Dispersionsmittel eingesetzt werden, mittels Polymerisation der Monomere. Bei dem Verfestigen in Schritt c) des erfindungsgemäßen Verfahrens wird vorzugsweise eine Festigkeit erreicht, die eine Handhabung des Rohlings erlaubt, beispielsweise um diesen aus der Gießform zu lösen oder ihn mittels CAD/CAM-Verfahren zu bearbeiten.

In einer bevorzugten Ausführungsform des erfindungsgemäßen Verfahrens umfasst dieses weiterhin einen Schritt d), in dem das in Schritt c) verfestigte Sediment gesintert wird. In den Fällen, in denen polymerisierbare Monomere als Dispersionsmittel eingesetzt werden, wird die gewünschte Festigkeit bereits durch die Polymerisation der Monomere erreicht. Diese kann beispielsweise unter Hochdruckbedingungen erfolgen, so dass der Schritt des Sinterns entfällt. In den Fällen, in denen ein Sinterschritt vorgesehen ist, kann das Sintern bis zu einer Festigkeit vorgenommen werden, die das weitere Bearbeiten des Rohlings mittels CAD/CAM-Verfahren erlaubt oder bis zu der Enddichte, die für seinen Einsatz als dentale Restauration angestrebt ist.

In einer weiterhin bevorzugten Ausführungsform umfasst das erfindungsgemäße Verfahren weiterhin einen Schritt des Infiltrierens des Rohlings. Bei der Infiltration wird der Rohling vorzugsweise mit einer flüssigen, härtbaren Zusammensetzung infiltriert, die nach Ende der Infiltration ausgehärtet wird. Der Schritt der Infiltration ist vor allem für die Fälle vorgesehen, in denen keine flüssigen polymerisierbaren Polymere als Dispersionsmittel verwendet werden. Die flüssige, härtbare Zusammensetzung umfasst vorzugsweise organische Monomere und/oder Präpolymere, vorzugsweise auf Basis von Acrylaten und/oder Methacrylaten. In einer weiteren bevorzugten Ausführungsform handelt es sich bei der flüssigen, härtbaren Zusammensetzung um ein Glas oder eine Glaskeramik. In einer Ausführungsform kann der Schritt des Infiltrierens nach dem Trocknen des Sediments vorgenommen werden. In einer anderen Ausführungsform kann dem Schritt des Infiltrierens ein Schritt des Sinterns vorgeschaltet werden, wobei beim Sintern eine Dichte des Rohlings angestrebt wird, die beispielsweise 65 bis 85% der theoretischen Dichte beträgt. Geeignete Verbindungen und Verfahren sind dem Fachmann aus WO 02/076907 und WO 2010/029515 bekannt.

In einer weiteren bevorzugten Ausführungsform handelt es sich bei der flüssigen, härtbaren Zusammensetzung um ein Glas oder eine Glaskeramik.

Im Rahmen der vorliegenden Erfindung wurde überraschend gefunden, dass Rohlinge für dentale Restaurationen mittels Sedimentation entsprechender Dispersionen erhalten werden können. Daher ist ein weiterer Gegenstand der vorliegenden Erfindung ein mittels des erfindungsgemäßen Verfahrens hergestellter Rohling, wobei der Rohling vorzugsweise einen Farbgradienten aufweist. In einer besonders bevorzugten Ausführungsform handelt es sich bei dem Rohling um einen Rohling, der mit einer flüssigen, härtbaren Zusammensetzung infiltriert ist. Weiterhin bevorzugt ist eine Ausführungsform, in der die härtbare Zusammensetzung ausgehärtet ist. In einer besonders bevorzugten Ausführungsform handelt es sich bei dem Rohling um einen Rohling aus einem Keramik-Polymer-Komposit mit interpenetrierenden Netzwerken, welches in der Dentalbranche auch als Hybridkeramik bezeichnet wird

Ein weiterer Gegenstand der vorliegenden Erfindung ist die Verwendung einer Suspension zur Herstellung eines Rohlings oder einer dentalen Restauration, wobei die Suspension ein flüssiges Dispersionsmittel, ein Basismaterial und ein oder mehrere färbende Substanzen umfasst, wobei sich der Rohling oder die dentale Restauration dadurch auszeichnet, dass er/sie einen Farbgradienten aufweist, der dem natürlichen Erscheinungsbild eines Zahn nachempfunden ist. Die verwendete Suspension ist vorzugsweise eine wie oben beschrieben.

Der erhaltene Rohling kann zu einer dentalen Restauration verarbeitet werden. Bei der dentalen Restauration, entweder erhalten durch Weiterverarbeitung des Rohlings oder direkt durch die erfindungsgemäße Verwendung der Suspension, handelt es sich vorzugsweise um Inlays, Onlays, Table Tops, Veneers, Kronen, Teilkronen, implantatgetragener Zahnersatz oder Brücken.

Beispiele:

Die vorliegende Erfindung soll anhand der folgenden Beispiele näher beschrieben werden, wobei diese keinesfalls als Einschränkung des Erfindungsgedanken zu verstehen sind. Die Angaben in Vol.-% beziehen sich jeweils auf das Gesamtvolumen der Suspension.

1. Steuerung des Farbverlaufs über die Partikelgröße

Ungefärbtes Feldspat-Pulver mit einer Partikelgröße D50 von 3 μm wurde mit roten und gelben anorganischen Pigmenten mit jeweils einer mittleren Partikelgröße D50 von 6 μm und einem Weißpigment mit einer mittleren Partikelgröße D50 von 1 μm vermengt. Die Mischung wurde mit Hilfe einer Kugelmühle in Wasser, einem nichtionischen Tensid und Wasserglas dispergiert, wobei der Feststoffgehalt der erhaltenen Suspension 53 Vol.-% betrug. Die Suspension wurde in eine Kunststoffgießform ausgebracht, sedimentiert und getrocknet. Das getrocknete Sediment wurde zu einem Formkörper gesintert.

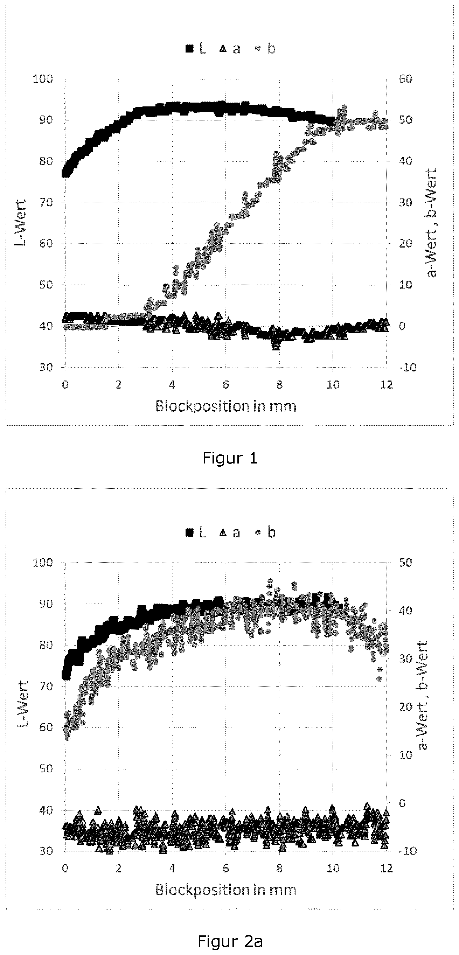

Die Farbunterschiede zwischen dem oberen und dem unteren Bereich des erhaltenen Rohlings waren deutlich ausgeprägt. Nach einem Bereich konstanter Weißfärbung im oberen Bereich des Blocks nahm die Gelbfärbung stufenlos und konstant entlang der Sedimentationsrichtung zu. Figur 1 zeigt die Lab-Farbwerte entlang der Sedimentationsrichtung.

Der Farbverlauf der Rohlinge wurde unter Verwendung der Lab-Werte (L*a*b) geprüft. Dazu wurden aus den erhaltenen Proben Scheiben mit einer Dicke von 3 mm gesägt und von diesen auf einem schwarzen Hintergrund positionierten Scheiben wurden Aufnahmen mit einem Lichtmikroskop gemacht. Mit Hilfe eines entsprechenden Bildverarbeitungsprogramms wurden daraus dann die Farbwerte entlang eines Profils ermittelt.

2. Steuerung des Farbverlaufs durch Feststoffgehalt

Ungefärbtes Feldspat-Pulver mit einer Partikelgröße D50 von 3 μm wurde mit einem roten und einem gelben Pigment, die ebenfalls eine Partikelgröße D50 von 3 μm aufwiesen, und einem Weißpigment mit einer Partikelgröße D50 von 1 μm vermengt. Die Mischung wurde in Wasser, einem nichtionischen Tensid und Wasserglas mit Hilfe einer Kugelmühle dispergiert. Auf diese Weise wurden Suspensionen mit einem Feststoffgehalt von 52 Vol.-%, 50 Vol.-% und 46 Vol.-% hergestellt.

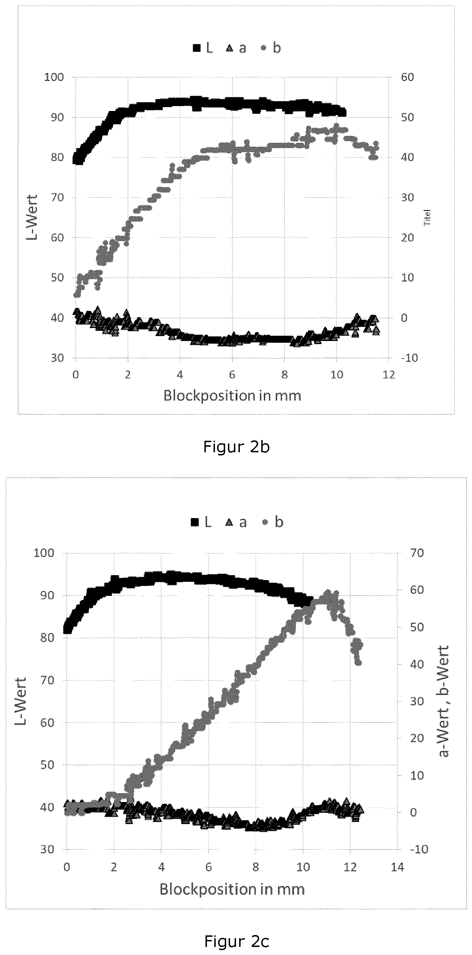

Figur 2a bis c zeigt die Lab-Werte der aus den Suspensionen durch Sedimentieren, Trocknen und Sintern erhaltenen Formkörper entlang der Sedimentationsrichtung. Wie aus den Proben ersichtlich, nahm der Farbgradient des Formkörpers mit abnehmendem Feststoffgehalt der Suspension zu. Somit lässt sich die Färbung der Rohlinge auf diese Weise von monochrom zu stufenlos multichrom einstellen.

3. Oberflächenmodifizierte Pigmente

Ein rotes, ein gelbes und ein weißes Pigment wurden jeweils mit einem Pulver auf Silikat-Basis vermengt und einer thermischen Behandlung unterzogen, bei der das Pulver auf Silikat-Basis und die Pigmente zu einer Fritte verschmolzen wurden, die dann mittels Mahlen auf eine mittlere Partikelgröße von 5 μm zerkleinert wurde.

Die so erhaltenen Pulver wurden zusammen mit einem ungefärbten Feldspat-Pulver mit einer mittleren Partikelgröße D50 von 3 μm vermengt und die Mischung mit Hilfe einer Kugelmühle in Wasser, einem nichtionischen Tensid und Wasserglas dispergiert. Aus den so hergestellten Suspensionen wurden durch Sedimentation, Trocknung und Sintern Formkörper gewonnen, deren Farbverlauf analysiert wurde.

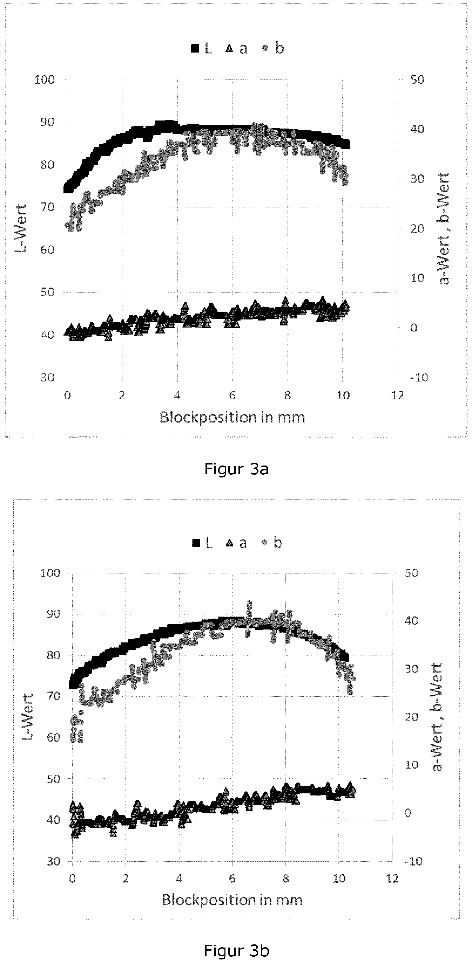

Figur 3a und b zeigt die Lab-Farbwerte der Rohlinge in Sedimentationsrichtung, wobei der Feststoffgehalt der Suspension 50 Vol.-% (Figur 3a) und 48 Vol.-% (Figur 3b) betrug. Wie aus den zur Verfügung gestellten Daten ersichtlich, kann durch die Verkapselung der Pigmente eine deutlich feinere Abstufung des Farbgradienten erreicht werden. Neben einer kontinuierlichen Steigerung des Gelbanteils konnte auch die Rotfärbung gezielt entlang der Sedimentationsrichtung stufenlos erhöht werden.

4. Vorgefärbtes Basismaterial

Ein rotes, ein gelbes und ein weißes Pigment wurden zusammen mit dem ungefärbten Basismaterial vermengt und unter Erhalt einer Fritte thermisch behandelt. Die Fritte wurde mittels Mahlens auf die gewünschte Partikelgröße zerkleinert und mit Hilfe einer Kugelmühle in Wasser, einem nichtionischen Tensid und Wasserglas dispergiert. Die Suspensionen mit einem Feststoffgehalt von 52 Vol.-%, 48 Vol.-% und 46 Vol.-% wurden sedimentiert und das Sediment getrocknet und gesintert. Der Farbgradient der so erhaltenen Formkörper wurde entlang der Sedimentationsrichtung vermessen.

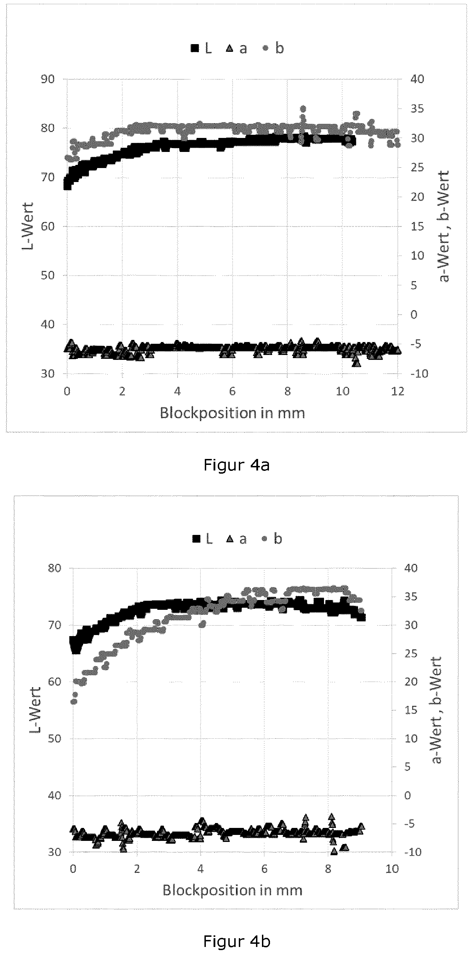

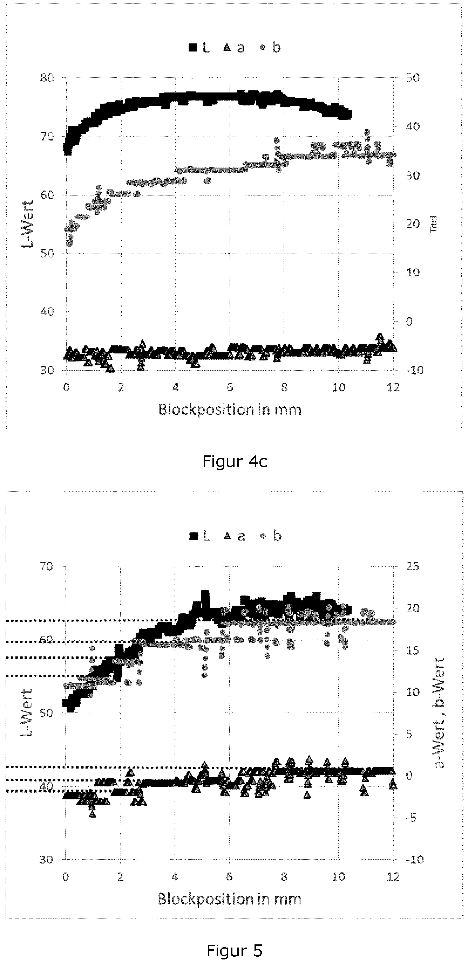

Figur 4a zeigt die Lab-Werte des Rohlings, der aus der Suspension mit einem Feststoffgehalt von 52 Vol.-% gewonnen wurde, Figur 4b den, der aus der Suspension mit einem Feststoffgehalt von 48 Vol.-% gewonnen wurde und Figur 4c den, der aus der Suspension mit einem Feststoffgehalt von 46 Vol.-% gewonnen wurde.

Wie den zur Verfügung gestellten Daten zu entnehmen ist, können auf diese Weise Rohlinge mit einem sehr feinen Farbgradienten erhalten werden.

5. Vergleichsbeispiel

Ein Formkörper wurde nach im Stand der Technik üblichen Verfahren hergestellt, indem vier Mischungen aus einem ungefärbten Basismaterial und unterschiedlichen Konzentrationen an Rotpigment, Gelbpigment und Weißpigment hergestellt wurden. Aus diesen Mischungen wurden mittels Sprühtrocknung vier Pressgranulate hergestellt, die mittels uniaxialem Mehrschichtpressen in einem 4-Kammer-Füllschuh verpresst wurden. Der so erhaltene Formkörper wurde entbindert und gesintert und der Farbgradient vermessen. Wie aus den in Figur 5 gezeigten Lab-Werten ersichtlich, sind die sprunghaften Änderungen der Farbwerte trotz der Verwendung von dünnen Schichten deutlich sichtbar, die das natürliche Erscheinungsbild des Restaurationsmaterials stören.

**Patentansprüche**

1. Verfahren zur Herstellung eines Rohlings für dentale Restaurationen mit Farbverlauf umfassend die Schritte:

   a) Bereitstellen einer Suspension umfassend

   i) flüssiges Dispersionsmittel,
   ii) ein Basismaterial und
   iii) ein oder mehrere färbende Substanzen;

   b) Sedimentation unter Ausbildung eines Farbgradienten im Sediment
   c) Verfestigen des Sediments unter Ausbildung eines Rohlings mit Farbverlauf.

2. Verfahren gemäß wenigstens einem der vorangehenden Ansprüche, **dadurch gekennzeichnet, dass** das Basismaterial ausgewählt ist aus der Gruppe bestehend aus Silikat-Glaskeramiken und -Gläsern, Aluminiumoxide, Spinelle und Zirkoniumoxide.

3. Verfahren gemäß wenigstens einem der vorangehenden Ansprüche, **dadurch gekennzeichnet, dass** die färbenden Substanzen aus Oxiden der Elemente Zr, Hf, Zn, Ti, Fe, Al, Si, Cr, Sb, Mn, Cd, Sn, Ca, Na, K, Mg, Ni, Co, Sc, Y, Ce, Er, Yb, Pr, Eu, Dy, Tb und Mischungen davon ausgewählt sind.

4. Verfahren gemäß wenigstens einem der vorangehenden Ansprüche, **dadurch gekennzeichnet, dass** die färbenden Substanzen in kolloidaler Form, Pulverform oder als Fritte vorliegen.

5. Verfahren gemäß wenigstens einem der vorangehenden Ansprüche, **dadurch gekennzeichnet, dass** das Dispersionsmittel ausgewählt ist aus der Gruppe bestehend aus Wasser, organische Lösungsmittel, insbesondere Alkohole, flüssige polymerisierbare Monomere und Mischungen hiervon.

6. Verfahren gemäß wenigstens einem der vorangehenden Ansprüche, **dadurch gekennzeichnet, dass** die Suspension einen Feststoffgehalt von 15 bis 65 Vol.-%, vorzugsweise 35 bis 60 Vol.-% aufweist, jeweils bezogen auf das Gesamtvolumen der Suspension.

7. Verfahren gemäß wenigstens einem der vorangehenden Ansprüche, **dadurch gekennzeichnet, dass** das Basismaterial eine zu den färbenden Substanzen abweichende Dichte aufweist, wobei die Dichte des Basismaterials vorzugsweise 2 bis 6 $g/cm^3$, vorzugsweise 2 bis 3 $g/cm^3$ beträgt und/oder die Dichte der ein oder mehreren färbenden Substanzen 3 bis 13 $g/cm^3$, vorzugsweise 4 bis 8 $g/cm^3$ beträgt.

8. Verfahren gemäß mindestens einem der vorangehenden Ansprüche, **dadurch gekennzeichnet, dass** das Basismaterial und/oder die färbenden Substanzen eine durchschnittliche Partikelgröße D50 von 0,03 bis 100 $\mu$m, vorzugsweise 1 bis 20 $\mu$m aufweisen, bestimmt mittels statischer und /oder dynamischer Lichtstreuung.

9. Verfahren gemäß wenigstens einem der vorangehenden Ansprüche, **dadurch gekennzeichnet, dass** das Basismaterial folgende Partikelgrößenverteilung aufweisen: einen D10 von 0,2*D50, vorzugsweise 0,5*D50 und einen D90 von 4*D50, vorzugsweise 2*D50.

10. Verfahren gemäß wenigstens einem der vorangehenden Ansprüche, **dadurch gekennzeichnet, dass** die Farbpigmentpartikel eine Partikelgrößenverteilung von D10 von 0,1*D50, vorzugsweise 0,4*D50 und einen D90 von 5*D50, vorzugsweise 2*D50 aufweisen.

11. Verfahren gemäß wenigstens einem der vorangehenden Ansprüche, **dadurch gekennzeichnet, dass** die Suspension weiterhin Additive umfasst, vorzugsweise Netzmittel und/oder Dispergatoren.

12. Verfahren gemäß wenigstens einem der vorangehenden Ansprüche, **dadurch gekennzeichnet, dass** das erfindungsgemäße Verfahren weiterhin einen Schritt des Infiltrierens des Rohlings umfasst.

13. Rohling zur Herstellung dentaler Restaurationen erhältlich nach einem Verfahren gemäß wenigstens einem der Ansprüche 1 bis 12.

14. Verwendung einer Suspension zur Herstellung einer dentalen Restauration mit Farbgradient, **dadurch gekennzeichnet, dass** die Suspension folgendes umfasst:

    i) flüssiges Dispersionsmittel,
    ii) ein Basismaterial und
    iii) ein oder mehrere färbende Substanzen.

15. Verwendung gemäß wenigstens einem der vorangehenden Ansprüche, **dadurch gekennzeichnet, dass** es sich bei der dentalen Restauration um ein Inlay, Onlay, Table Top, Veneer, Kronen, Teilkronen, implantatgetragener Zahnersatz oder eine Brücke handelt.

Figur 1

Figur 2a

Figur 2b

Figur 2c

Figur 3a

Figur 3b

Figur 4a

Figur 4b

Figur 4c

Figur 5

**Europäisches Patentamt**
**European Patent Office**
**Office européen des brevets**

# EUROPÄISCHER RECHERCHENBERICHT

Nummer der Anmeldung

EP 21 17 4713

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2019/381769 A1 (REINSHAGEN JÖRG [DE] ET AL) 19. Dezember 2019 (2019-12-19) | 13 | INV.<br>C04B35/111 |
| Y | * Anspruch 22; Beispiel 8 *<br>----- | 12 | C04B35/16<br>C04B35/443 |
| X | US 2012/308837 A1 (SCHLECHTRIEMEN NADJA [AT] ET AL) 6. Dezember 2012 (2012-12-06)<br>* Absätze [0058], [0059], [0075], [0076], [0109], [0110] *<br>----- | 13-15 | C04B35/486<br>C04B35/626<br>A61C13/00<br>C03C10/00 |
| X | US 2019/247168 A1 (OLDENBURGER DANIEL [DE] ET AL) 15. August 2019 (2019-08-15)<br>* Absatz [0070] - Absatz [0071]; Abbildung 2 *<br>----- | 13 | |
| X | WO 2020/231329 A1 (AGENCY SCIENCE TECH & RES [SG]) 19. November 2020 (2020-11-19) | 1,3-8, 11,13-15 | |
| Y | * Seite 7, Zeile 9 - Seite 8, Zeile 21; Abbildung 1; Beispiele 1-3 *<br>* Seite 10, Zeile 10 - Zeile 32 *<br>----- | 9,10,12 | |
| X | US 2018/072628 A1 (CORNELL DONALD F [US] ET AL) 15. März 2018 (2018-03-15) | 1-8,11, 13-15 | **RECHERCHIERTE SACHGEBIETE (IPC)** |
| Y | * Anspruch 7; Abbildungen 1-3; Beispiele 1-8 *<br>----- | 9,10,12 | C04B<br>A61C<br>A61K |
| Y | WO 2015/055950 A1 (SAINT GOBAIN CT RECHERCHES [FR]) 23. April 2015 (2015-04-23)<br>* Seite 1, Absatz 1 - Absatz 3; Ansprüche 1-5 *<br>----- | 9,10 | C03C |

Der vorliegende Recherchenbericht wurde für alle Patentansprüche erstellt

| Recherchenort | Abschlußdatum der Recherche | Prüfer |
|---|---|---|
| Den Haag | 7. Oktober 2021 | Raming, Tomas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 21 17 4713

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-10-2021

| Im Recherchenbericht angeführtes Patentdokument | Datum der Veröffentlichung | Mitglied(er) der Patentfamilie | Datum der Veröffentlichung |
|---|---|---|---|
| US 2019381769 A1 | 19-12-2019 | CN 109937140 A | 25-06-2019 |
| | | EP 3558671 A1 | 30-10-2019 |
| | | JP 6893556 B2 | 23-06-2021 |
| | | JP 2020500191 A | 09-01-2020 |
| | | JP 2021152028 A | 30-09-2021 |
| | | KR 20190100236 A | 28-08-2019 |
| | | US 2019381769 A1 | 19-12-2019 |
| | | WO 2018115529 A1 | 28-06-2018 |
| US 2012308837 A1 | 06-12-2012 | EP 2529694 A1 | 05-12-2012 |
| | | JP 2012250022 A | 20-12-2012 |
| | | US 2012308837 A1 | 06-12-2012 |
| US 2019247168 A1 | 15-08-2019 | DE 102018103415 A1 | 22-08-2019 |
| | | EP 3527166 A1 | 21-08-2019 |
| | | US 2019247168 A1 | 15-08-2019 |
| WO 2020231329 A1 | 19-11-2020 | SG 10201904398P A | 30-12-2020 |
| | | WO 2020231329 A1 | 19-11-2020 |
| US 2018072628 A1 | 15-03-2018 | US 2018072628 A1 | 15-03-2018 |
| | | WO 2018049331 A1 | 15-03-2018 |
| WO 2015055950 A1 | 23-04-2015 | FR 3012135 A1 | 24-04-2015 |
| | | WO 2015055950 A1 | 23-04-2015 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2020025795 A
- DE 19714178
- EP 1900341 A
- EP 3215820 A
- EP 3527166 A
- WO 02076907 A
- WO 2010029515 A